(19) Europäisches Patentamt
European Patent Office
Office européen des brevets

(11) Veröffentlichungsnummer : **0 405 240 B1**

# (12) EUROPÄISCHE PATENTSCHRIFT

(45) Veröffentlichungstag der Patentschrift :
**06.10.93 Patentblatt 93/40**

(51) Int. Cl.$^5$ : **A01N 43/653,** // C07D249/08

(21) Anmeldenummer : **90111257.3**

(22) Anmeldetag : **14.06.90**

## (54) Verwendung von substituierten Bis-azolyl-Derivaten zur Bekämpfung von Pilzen im Pflanzenschutz.

(30) Priorität : **28.06.89 DE 3921162**

(43) Veröffentlichungstag der Anmeldung :
**02.01.91 Patentblatt 91/01**

(45) Bekanntmachung des Hinweises auf die Patenterteilung :
**06.10.93 Patentblatt 93/40**

(84) Benannte Vertragsstaaten :
**BE CH DE FR GB IT LI NL**

(56) Entgegenhaltungen :
**EP-A- 0 180 838**
**EP-B- 0 164 246**
**DE-A- 3 440 114**
**DE-A- 3 608 792**

(73) Patentinhaber : **BAYER AG**
**D-51368 Leverkusen (DE)**

(72) Erfinder : **Stroech, Klaus, Dr.**
**Rolsberger Strasse 22**
**D-5650 Solingen 19 (DE)**
Erfinder : **Backens-Hammerschmidt, Susanne, Dr.**
**Kicke 19**
**D-5060 Bergisch-Gladbach 1 (DE)**
Erfinder : **Hänssler, Gerd, Dr.**
**Am Arenzberg 58a**
**D-5090 Leverkusen 3 (DE)**
Erfinder : **Dutzmann, Stefan, Dr.**
**Leinenweberweg 33**
**D-4000 Düsseldorf 13 (DE)**

EP 0 405 240 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von substituierten Bis-azolyl-Derivaten zur Bekämpfung von phytopathogenen Pilzen.

Es ist bereits bekannt geworden, daß bestimmte Bis-azolyl-Derivate als Fungizide im Pflanzenschutz geeignet sind (vgl. EP-OS 0 180 838). So lassen sich zum Beispiel 1-(4-Chlorphenyl)-1-[1-(1,2,4-triazol-1-yl)-cyclopropyl]-2-(1,2,4-triazol-1-yl)-1-ethanol und 1-(4-Methylphenyl)-1-[1-(1,2,4-triazol-1-yl)-cyclopropyl]-2-(1,2,4-triazol-1-yl)-1-ethanol zur Bekämpfung von Pilzen im Pflanzenschutz einsetzen. Die Wirkung dieser Stoffe ist gut; sie läßt allerdings bei niedrigen Aufwandmengen in manchen Fällen zu wünschen übrig.

Es wurde nun gefunden, daß die substituierten Bis-azolyl-Derivate der Formel

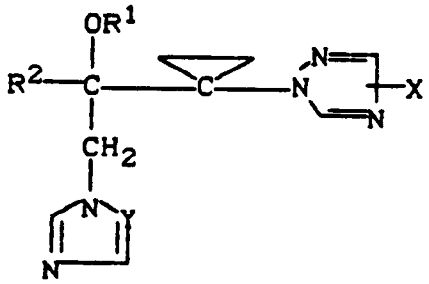

(I)

in welcher

$R^1$ - für Wasserstoff steht, oder
- für geradkettiges oder verzweigtes Alkyl mit bis zu 8 Kohlenstoffatomen steht, das gegebenenfalls durch Aryl substituiert ist, welches seinerseits durch Halogen, $C_1$-$C_8$-Alkyl, $C_1$-$C_8$-Alkoxy, $C_1$-$C_8$-Alkylthio, $C_1$-$C_8$-Halogenalkyl, $C_1$-$C_8$-Halogenalkoxy, $C_1$-$C_8$-Halogenalkylthio oder durch Phenyl substituiert ist, das gegebenenfalls bis zu 4-fach gleich oder verschieden durch Halogen substituiert ist oder
- für geradkettiges oder verzweigtes Alkenyl oder Alkinyl mit bis zu 8 Kohlenstoffatomen steht oder
- für Trialkylsilyl mit bis zu 8 Kohlenstoffatomen im Alkylteil steht oder
- für Alkylcarbonyl mit bis zu 8 Kohlenstoffatomen im Alkylteil steht,

$R^2$ - für Aryl mit 6 bis 10 Kohlenstoffatomen steht, das gegebenenfalls bis zu 4-fach gleich oder verschieden substituiert ist durch Halogen, $C_1$-$C_8$-Alkyl, $C_1$-$C_8$-Alkoxy, $C_1$-$C_8$-Alkylthio, Halogenalkyl mit 1 bis 8 Kohlenstoffatomen und 1 bis 5 gleichen oder verschiedenen Halogenatomen, Halogenalkoxy mit 1 bis 8 Kohlenstoffatomen und 1 bis 5 gleichen oder verschiedenen Halogenatomen, Halogenalkylthio mit 1 bis 8 Kohlenstoffatomen und 1 bis 5 gleichen oder verschiedenen Halogenatomen, gegebenenfalls ein- bis dreifach durch Halogen substituiertes Phenyl, gegebenenfalls ein- bis dreifach durch Halogen substituiertes Phenoxy und/oder durch gegebenenfalls ein- bis dreifach durch Halogen substituiertes Phenylthio, oder

$R^2$ - für einen 5- oder 6-gliedrigen Heterocyclus steht, der ein oder mehrere Heteroatome, wie Schwefel, Sauerstoff oder Stickstoff, enthalten kann und der gegebenenfalls durch Halogen, $C_1$-$C_8$-Alkyl, $C_1$-$C_8$-Alkoxy, $C_1$-$C_8$-Alkylthio, Halogenalkyl mit 1 bis 8 Kohlenstoffatomen und 1 bis 5 gleichen oder verschiedenen Halogenatomen, Halogenalkoxy mit 1 bis 8 Kohlenstoffatomen und 1 bis 5 gleichen oder verschiedenen Halogenatomen und/oder Halogenalkylthio mit 1 bis 8 Kohlenstoffatomen und 1 bis 5 gleichen oder verschiedenen Halogenatomen substituiert ist,

X - für Halogen steht,

und

Y - für ein Stickstoffatom oder für die CH-Gruppe steht,

sowie deren Säureadditions-Salze und Metallsalz-Komplexe sehr gut zur Bekämpfung von Pilzen im Pflanzenschutz geeignet sind.

Die erfindungsgemäß verwendbaren Stoffe enthalten ein asymmetrisch substituiertes Kohlenstoffatom. Sie können daher in optischen Isomerenformen anfallen. Die vorliegende Erfindung betrifft sowohl die Verwendung der einzelnen Isomere als auch von deren Gemischen.

Überraschenderweise besitzen die erfindungsgemäß verwendbaren Stoffe bei der Bekämpfung phytopathogener Pilze eine wesentlich bessere Wirksamkeit als 1-(4-Chlor-phenyl)-1-[1-(1,2,4-triazol-1-yl)-cyclopropyl]-2-(1,2,4-triazol-1-yl)-1-ethanol und 1-(4-Methyl-phenyl)-1-[1-(1,2,4-triazol-1-yl)-cyclopropyl]-2-(1,2,4-triazol-1-yl)-1-ethanol, welches konstitutionell ähnliche, vorbekannte Wirkstoffe gleicher Wirkungsrichtung sind.

Die erfindungsgemäß verwendbaren Stoffe sind durch die Formel (I) allgemein definiert. Vorzugsweise verwendbar sind diejenigen Stoffe der Formel (I), in denen

$R^1$ - für Wasserstoff steht oder
- für geradkettiges oder verzweigtes Alkyl mit bis zu 6 Kohlenstoffatomen steht, das gegebenenfalls durch Phenyl substituiert ist, welches seinerseits durch Halogen, $C_1$-$C_6$-Alkyl, $C_1$-$C_6$-Alkoxy, $C_1$-$C_6$-Alkylthio, $C_1$-$C_6$-Halogenalkyl oder durch Phenyl substituiert sein kann, das gegebenenfalls bis zu 3-fach gleich oder verschieden durch Halogen substituiert ist oder
- für geradkettiges oder verzweigtes Alkenyl oder Alkinyl mit bis zu 6 Kohlenstoffatomen steht oder
- für Trialkylsilyl mit bis zu 6 Kohlenstoffatomen in jedem Alkylteil steht oder
- für Alkylcarbonyl mit bis zu 6 Kohlenstoffatomen im Alkylteil steht,

$R^2$ - für Phenyl oder Naphthyl steht, wobei jeder dieser Reste einfach bis dreifach, gleichartig oder verschieden substituiert sein kann durch Halogen, $C_1$-$C_6$-Alkoxy, $C_1$-$C_6$-Alkylthio, Halogenalkyl mit 1 bis 6 Kohlenstoffatomen und 1 bis 5 gleichen oder verschiedenen Halogenatomen, Halogenalkoxy mit 1 bis 6 Kohlenstoffatomen und 1 bis 5 gleichen oder verschiedenen Halogenatomen, Halogenalkylthio mit 1 bis 6 Kohlenstoffatomen und 1 bis 5 gleichen oder verschiedenen Halogenatomen, gegebenenfalls ein- oder zweifach durch Fluor, Chlor und/oder Brom substituiertes Phenyl, gegebenenfalls ein- oder zweifach durch Fluor, Chlor und/oder Brom substituiertes Phenoxy und/oder durch gegebenenfalls ein- oder zweifach durch Fluor, Chlor und/oder Brom substituiertes Phenylthio, oder

$R^2$ - für einen 5- oder 6-gliedrigen Heterocyclus mit 1 bis 3 Sauerstoff-, Schwefel-, und/oder Stickstoffatomen steht, wobei jeder dieser Heterocyclen ein- oder zweifach, gleichartig oder verschieden substituiert sein kann durch Halogen, $C_1$-$C_6$-Alkyl, $C_1$-$C_6$-Alkoxy, $C_1$-$C_6$-Alkylthio, Halogenalkyl mit 1 bis 6 Kohlenstoffatomen und 1 bis 5 gleichen oder verschiedenen Halogenatomen, Halogenalkoxy mit 1 bis 6 Kohlenstoffatomen und 1 bis 5 gleichen oder verschiedenen Halogenatomen und/oder durch Halogenalkylthio mit 1 bis 6 Kohlenstoffatomen und 1 bis 5 gleichen oder verschiedenen Halogenatomen,

X - für Halogen steht,

und

Y - für ein Stickstoffatom oder für die CH-Gruppe steht.

Besonders bevorzugt verwendbar sind diejenigen Stoffe der Formel (I), in denen

$R^1$ - für Wasserstoff steht oder
- für geradkettiges oder verzweigtes Alkyl mit bis zu 4 Kohlenstoffatomen steht, das gegebenenfalls durch Phenyl substituiert ist, welches seinerseits durch Fluor, Chlor, Brom, Methyl, Methoxy, Methylthio, Trifluormethyl und/oder durch gegebenenfalls ein- oder zweifach durch Fluor, Chlor und/oder Brom substituiertes Phenyl substituiert ist, oder
- für geradkettiges oder verzweigtes Alkenyl oder Alkinyl mit jeweils bis zu 4 Kohlenstoffatomen steht,
- für Trimethylsilyl oder für Alkylcarbonyl mit 1 bis 4 Kohlenstoffatomen im Alkylteil steht,

$R^2$ - für Phenyl oder Naphthyl steht, wobei jeder dieser Reste einfach oder zweifach, gleichartig oder verschieden substituiert sein kann durch Fluor, Chlor, Brom, Methyl, Ethyl, n-Propyl, Isopropyl, n-Butyl, tert.-Butyl, Methoxy, Ethoxy, Methylthio, Ethylthio, Trifluormethyl, Trifluormethoxy, Trifluormethylthio, Phenyl, Phenoxy, Chlorphenyl, Chlorphenoxy, Fluorphenyl und/oder Fluorphenoxy, oder

$R^2$ - für einen 5- oder 6-gliedrigen Heterocyclus mit 1 bis 3 Sauerstoff-, Schwefel- und/oder Stickstoffatomen steht, wobei jeder dieser Heterocyclen ein- oder zweifach, gleichartig oder verschieden substituiert sein kann durch Fluor, Chlor, Brom, Methyl, Ethyl, Methoxy, Ethoxy, Methylthio, Trifluormethyl, Trifluormethoxy und/oder durch Trifluormethylthio,

X - für Fluor, Chlor oder Brom steht

und

Y - für ein Stickstoffatom oder für die CH-Gruppe steht.

Bevorzugt verwendbare erfindungsgemäße Verbindungen sind auch Additionsprodukte aus Säuren und denjenigen substituierten Bis-azolyl-Derivaten der Formel (I), in denen $R^1$, $R^2$, X und Y die Bedeutungen haben, die bereits vorzugsweise für diese Reste genannt wurden.

Zu den Säuren, die addiert werden können, gehören vorzugsweise Halogenwasserstoffsäuren wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren wie z.B. Essigsäure, Maleinsäure, Bernsteinsäure, Fumarsäure, Weinsäure, Zitronensäure, Salizylsäure, Sorbinsäure und Milchsäure sowie Sulfonsäuren wie z.B. p-Toluolsulfonsäure, 1,5-Naphthalindisulfonsäure oder Camphersulfonsäure.

Außerdem bevorzugt verwendbare erfindungsgemäße Verbindungen sind auch Additionsprodukte aus Salzen von Metallen der II. bis IV. Haupt- und der I. und II. sowie IV. bis VIII. Nebengruppe des Periodensystems

der Elemente und denjenigen substituierten Bis-azolyl-Derivaten der Formel (I), in denen $R^1$, $R^2$, X und Y die Bedeutungen haben, die bereits vorzugsweise für diese Reste genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren wie z.B. die Chlorwasserstoffsäure und die Bromwasserstoffsäure, ferner Phosphorsäure, Salpetersäure und Schwefelsäure.

Die erfindungsgemäß verwendbaren Stoffe sind Gegenstand eines gesonderten Schutzbegehrens. Sie lassen sich herstellen, indem man

a) in einer ersten Stufe Azolyl-cyclo-propylketone der Formel

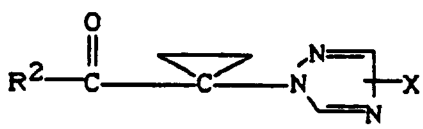

(II)

in welcher

$R^2$ und X die oben angegebene Bedeutung haben,

entweder

α) mit Dimethyloxosulfonium-methylid der Formel

$$\overset{\delta\oplus\,\delta\ominus}{(CH_3)_2SOCH_2} \qquad (III)$$

oder

β) mit Dimethylsulfonium-methylid der Formel

$$\overset{\delta\oplus\,\delta\ominus}{(CH_3)_2S\ CH_2} \qquad (IV)$$

in Gegenwart eines Verdünnungsmittels umsetzt, und die dabei entstehenden Azolyl-cyclo-propyl-oxirane der Formel

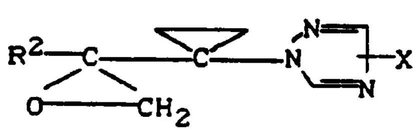

(V)

in welcher

$R^2$ und X die oben angegebene Bedeutung haben,

in einer zweiten Stufe mit Azolen der Formel

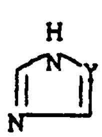

(VI)

in welcher

Y die oben angegebene Bedeutung hat,
in Gegenwart eines Verdünnungsmittels und in Gegenwart einer Base umsetzt oder
b) Bis-azolyl-keto-Derivate der Formel

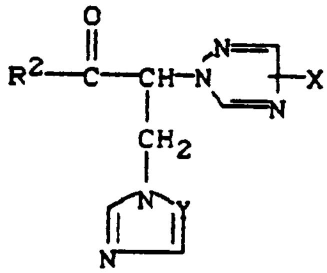

(VII)

in welcher
$R^2$, X und Y die oben angegebene Bedeutung haben,
mit Dimethyloxosulfonium-methylid der Formel

$$\overset{\delta^{\oplus}\,\delta^{\ominus}}{(CH_3)_2SOCH_2} \quad (III)$$

in Gegenwart eines Verdünnungsmittels umsetzt,
oder
c) Hydroxy-Verbindungen der Formel

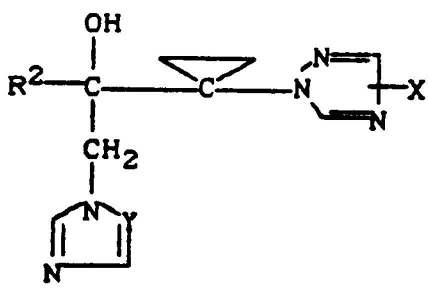

(I a)

in welcher
$R^2$, X und Y die oben angegebene Bedeutung haben,
mit Basen in Gegenwart eines Verdünnungsmittels umsetzt und die dabei entstehenden Alkoholate der Formel

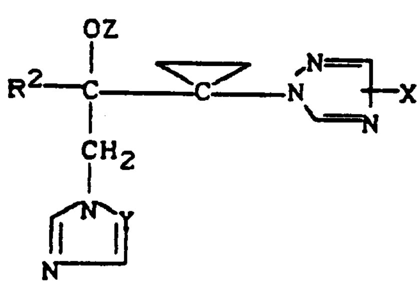

(I b)

in welcher
$R^2$, X und Y die oben angegebene Bedeutung haben, und
Z für einen Base-Rest steht,

mit Halogenverbindungen der Formel

$R^3$-Hal (VIII)

in welcher

$R^3$ - für geradkettiges oder verzweigtes Alkyl mit bis zu 8 Kohlenstoffatomen steht, das gegebenenfalls durch Aryl substituiert ist, welches seinerseits durch Halogen, $C_1$-$C_8$-Alkyl, $C_1$-$C_8$-Alkoxy, $C_1$-$C_8$-Alkylthio, $C_1$-$C_8$-Halogenalkyl, $C_1$-$C_8$-Halogenalkoxy, $C_1$-$C_8$-Halogenalkylthio oder durch Phenyl substituiert ist, das gegebenenfalls bis zu 4-fach gleich oder verschieden durch Halogen substituiert ist oder
- für geradkettiges oder verzweigtes Alkenyl oder Alkinyl mit bis zu 8 Kohlenstoffatomen steht oder
- für Trialkylsilyl mit bis zu 8 Kohlenstoffatomen im Alkylteil steht oder
- für Alkylcarbonyl mit bis zu 8 Kohlenstoffatomen im Alkylteil steht,

und

Hal für Chlor, Brom oder Iod steht,

in Gegenwart eines Verdünnungsmittels umsetzt;

und gegebenenfalls anschließend an die so erhaltenen Verbindungen der Formel (I) eine Säure oder ein Metallsalz addiert.

Die bei dem Verfahren a) als Ausgangsstoffe benötigten Azolyl-cyclopropyl-ketone der Formel (II) lassen sich herstellen, indem man Halogen-propyl-ketone der Formel

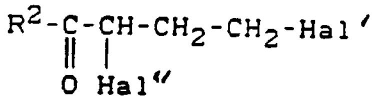

(IX)

in welcher

$R^2$ die oben angegebene Bedeutung hat und

Hal' und Hal" gleich oder verschieden sind und für Chlor oder Brom stehen,

mit Triazolen der Formel

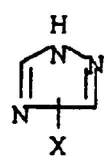

(X)

in welcher

X die oben angegebene Bedeutung hat,

in Gegenwart eines Verdünnungsmittels und in Gegenwart einer Base umsetzt.

Als Verdünnungsmittel kommen für die Herstellung der Ketone der Formel (II) unter den Reaktionsbedingungen inerte organische Lösungsmittel in Frage. Hierzu gehören vorzugsweise Alkohole wie z.B. Ethanol, Methoxyethanol oder Propanol, Ketone wie z.B. Aceton und 2-Butanon, Nitrile wie z.B. Acetonitril, Ester wie z.B. Essigester, Ether wie z.B. Dioxan, aromatische Kohlenwasserstoffe wie z.B. Benzol und Toluol oder Amide wie z.B. Dimethylformamid.

Als Basen kommen für diese Umsetzung alle üblicherweise verwendbaren anorganischen oder organischen Basen in Betracht. Hierzu gehören vorzugsweise Alkalicarbonate wie z.B. Natrium- und Kaliumcarbonat, Alkalihydroxide wie z.B. Natriumhydroxid, Alkalialkoholate wie z.B. Natrium- und Kalium-methylat und -ethylat, Alkalihydride wie z.B. Natriumhydrid sowie niedere tertiäre Alkylamine, Cycloalkylamine und Aralkylamine, wie insbesondere Triethylamin.

Die Reaktionstemperaturen können bei der Durchführung dieser Umsetzung in einem größeren Bereich variiert werden. Im allgemeinen arbeitet man bei Temperaturen zwischen 0°C und 200°C, vorzugsweise zwischen 60°C und 150°C.

Bei der Durchführung dieser Umsetzung setzt man auf 1 Mol an Halogen-propyl-keton der Formel (IX) im allgemeinen 1 bis 2 Mol Triazol der Formel (X) und gegebenenfalls 1 bis 2 Mol an Base ein. Die Isolierung der Azolyl-cyclopropyl-ketone der Formel (II) erfolgt nach üblichen Methoden.

Die Halogen-propyl-ketone der Formel (IX) sind bekannt oder lassen sich nach üblichen Methoden in einfacher Weise herstellen (vgl. DE-OS 2 521 104, DE-OS 2 320 355 und DE-OS 2 351 948).

Das wowohl bei dem Verfahren (a), Variante $\alpha$) als auch bei dem Verfahren (b) als Reaktionskomponente

benötigte Dimethyloxosulfonium-methylid der Formel (III) ist bekannt (vgl. J. Am. Chem. Soc. 87, 1363 - 1364 (1965)). Es wird bei den obigen Umsetzungen im frisch zubereiteten Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Das bei dem Verfahren (a, Variante β) als Reaktionskomponente benötigte Dimethylsulfonium-methylid der Formel (IV) ist ebenfalls bekannt (vgl. Heterocycles 8, (1977) 397). Es wird bei der obigen Umsetzung ebenfalls im frisch zubereiteten Zustand verwendet.

Die für die zweite Stufe des Verfahrens (a) als Ausgangsstoffe benötigten Azole der Formel (VI) sind allgemein bekannte Verbindungen der organischen Chemie.

Die bei dem Verfahren (b) als Ausgangsstoffe benötigten Bis-azolyl-keto-Derivate der Formel (VII) lassen sich herstellen, indem man Azolyl-ketone der Formel

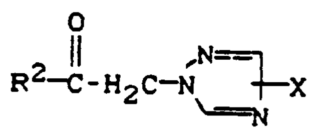

(XI)

in welcher
$R^2$ und X die oben angegebene Bedeutung haben,
mit Hydroxymethylazolen der Formel

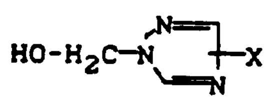

(XII)

in welcher
Y die oben angegebene Bedeutung hat,
in Gegenwart eines Katalysators und in Gegenwart eines Verdünnungsmittels umsetzt.

Als Verdünnungsmittel kommen für dieses Verfahren zur Herstellung der Bis-azolyl-keto-Derivate der Formel (VII) vorzugsweise inerte organische Lösungsmittel in Frage. Hierzu gehören vorzugsweise Alkohole, wie Methanol und Ethanol, Ether, wie Tetrahydrofuran und Dioxan, aromatische Kohlenwasserstoffe, wie Benzol und Toluol, sowie halogenierte aliphatische und aromatische Kohlenwasserstoffe, wie Methylenchlorid, Chloroform, Chlorbenzol und Dichlorbenzol.

Dieses Verfahren wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren wie z.B. Bortrifluorid, Bortrichlorid, Zinntetrachlorid oder Titantetrachlorid, organische Basen wie Pyridin und Piperidin sowie insbesondere Piperidinacetat.

Die Reaktionstemperaturen können bei der Durchführung dieses Verfahrens in einem größeren Bereich variiert werden. Im allgemeinen arbeitet man zwischen 20 und 160°C, vorzugsweise bei der Siedetemperatur des jeweiligen Lösungsmittels.

Bei der Durchführung des Verfahrens zur Herstellung der Bis-azolyl-keto-Derivate der Formel (VII) setzt man auf 1 Mol an Azolyl-keton der Formel (XI) 1 bis 1,5 Mol an Hydroxymethylazol der Formel (XII) und katalytische bis 0,2 molare Mengen an Katalysator ein.

Die Azolyl-ketone der Formel (XI) sind bekannt (vgl. DE-OS 2 431 407, DE-OS 2 610 022 und DE-OS 2 638 470).

Die Hydroxymethylazole der Formel (XII) sind ebenfalls bekannt (vgl. EP-OS 0 006 102 sowie Chem. Heterocycl. Comp. 1980, 189).

Die für das Verfahren (c) als Ausgangsstoffe zu verwendenden Hydroxy-Verbindungen der Formel (I a) sind erfindungsgemäß verwendbare Verbindungen. Ihre Überführung in Alkoholate der Formel (I b) erfolgt mit Hilfe von starken Basen. Vorzugsweise verwendbar sind hierbei Alkalimetall-amide wie Natriumamid und Kaliumamid, ferner Alkalimetallhydride wie Natriumhydrid, quarternäre Ammoniumhydroxide und Phosphoniumhydroxide. Z steht deshalb in den Verbindungen der Formel (I b) vorzugsweise für Natrium, Kalium, quarternäres Ammonium oder Phosphonium.

Als Verdünnungsmittel kommen bei der Umsetzung der Verbindungen der Formel (I a) zu den Alkoholaten der Formel (I b) inerte organische Solventien in Betracht. Vorzugsweise verwendbar sind Ether wie Dioxan.

Die Umsetzung der Verbindungen der Formel (I a) zu den Alkoholaten der Formel (I b) erfolgt vorzugsweise

bei Raumtemperatur.

Die bei dem Verfahren (c) als Reaktionskomponenten benötigten Halogenverbindungen sind durch die Formel (VIII) algemein definiert. In dieser Formel steht $R^3$ vorzugsweise für die Bedeutungen, die bereits im Zusammenhang mit der Beschreibung der erfindungsgemäß verwendbaren Stoffe der Formel (I) für den Substituenten $R^1$ vorzugsweise genannt wurden, mit Ausnahme der Bedeutung von Wasserstoff. Hal steht für Chlor, Brom oder Iod.

Die Halogenverbindungen der Formel (VIII) sind allgemein bekannte Verbindungen der organischen Chemie.

Als Verdünnungsmittel kommen für die erste Stufe des Verfahrens (a) inerte organische Lösungsmittel in Frage. Hierzu gehören vorzugsweise Ether, wie Tetrahydrofuran oder Dioxan, aliphatische und aromatische Kohlenwasserstoffe, wie insbesondere Benzol, Toluol oder Xylol sowie Dimethylsulfoxid.

Die Reaktionstemperaturen können bei der Durchführung der ersten Stufe des Verfahrens (a) in einem größeren Bereich variiert werden. Im allgemeinen arbeitet man zwischen 0 und 100°C, vorzugsweise zwischen 10 und 60°C.

Bei der Durchführung der ersten Stufe des Verfahrens (a) setzt man auf 1 Mol Azolyl-cyclopropyl-keton der Formel (II) vorzugsweise 1 bis 3 Mol Dimethyloxosulfonium-methylid der Formel (III), in situ erzeugt aus Trimethyloxosulfoniumiodid in Dimethylsulfoxid und Natriumhydrid, oder Dimethylsulfonium-methylid der Formel (V) ein.

Die Isolierung der Zwischenprodukte der Formel (V) erfolgt in allgmein üblicher Art und Weise.

Als Verdünnungsmittel kommen für die zweite Stufe des Verfahrens (a) inerte organische Lösungsmittel in Frage. Vorzugsweise verwendbar sind Nitrile, wie insbesondere Acetonitril, aromatische Kohlenwasserstoffe, wie Benzol, Toluol und Dichlorbenzol, Formamide, wie insbesondere Dimethylformamid, sowie Hexamethylphosphorsäuretriamid.

Die zweite Stufe des Verfahrens (a) wird in Gegenwart einer Base durchgeführt. Dabei kommen alle üblicherweise verwendbaren anorganischen und organischen Basen in Betracht. Hierzu gehören vorzugsweise Alkalicarbonate wie z.B. Natrium- und Kaliumcarbonat, Alkalihydroxide wie z.B. Natriumhydroxid, Alkalialkoholate wie z.B. Natrium- und Kalium-methylat und -ethylat, Alkalihydride wie z.B. Natriumhydrid sowie niedere tertiäre Alkylamine, Cycloalkylamine und Aralkylamine wie insbesondere Triethylamin.

Die Reaktionstemperaturen können bei der Durchführung der zweiten Stufe des Verfahrens (a) in einem größeren Bereich variiert werden. Im allgemeinen arbeitet man bei Temperaturen zwischen 0°C und 200°C, vorzugsweise zwischen 60°C und 150°C.

Bei der Durchführung der zweiten Stufe des Verfahrens (a) setzt man vorzugsweise 1 Mol Oxiran der Formel (V), 1 bis 2 Mol Azol der Formel (VI) und 1 bis 2 Mol Base ein. Die Isolierung der Endprodukte erfolgt in allgemein üblicher Weise.

Die Reaktionsbedingungen bei der Durchführung des Verfahrens (b) entsprechen denen für die Durchführung der ersten Stufe des Verfahrens (a).

Bei der Umsetzung von Alkoholaten der Formel (Ib) mit Halogenverbindungen der Formel (VIII) nach dem Verfahren (c) kommen als Verdünnungsmittel inerte organische Lösungsmittel in Frage. Hierzu gehören vorzugsweise Ether, wie Diethylether oder Dioxan, aromatische Kohlenwasserstoffe, wie Benzol, in einzelnen Fällen auch chlorierte Kohlenwasserstoffe, wie Chloroform, Methylenchlorid oder Tetrachlorkohlenstoff, sowie Hexamethylphosphorsäuretriamid.

Die Reaktionstemperaturen können bei der Umsetzung von Alkoholaten der Formel (I b) mit Halogenverbindungen der Formel (VIII) nach dem Verfahren (c) in einem größeren Bereich variiert werden. Im allgemeinen arbeitet man bei Temperaturen zwischen 0°C und 120°C, vorzugsweise zwischen 20°C und 100°C.

Bei der Durchführung des Verfahrens (c) setzt man auf 1 Mol eines Alkoholates der Formel (I b) vorzugsweise 1 bis 2 Mol einer Halogenverbindung der Formel (VIII) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt und in üblicher Weise aufgearbeitet und gereingt.

In einer bevorzugten Ausführungsform wird bei dem Verfahren (c) zweckmäßigerweise so vorgegangen, daß man von einer Hydroxy-Verbindung der Formel (I a) ausgeht, letztere in einem geeigneten organischen Lösungsmittel mittels Alkalimetall-hydrid oder Alkalimetall-amid in ein Alkalimetallalkoholat der Formel (I b) überführt und letzteres ohne Isolierung sofort mit einer Halogenverbindung der Formel (VIII) umsetzt, wobei unter Austritt von Alkalihalogenid die erfindungsgemäßen Verbindungen der Formel (I) in einem Arbeitsgang erhalten werden.

Nach einer weiteren bevorzugten Ausführungsform des Verfahrens (c) werden zweckmäßigerweise die Herstellung der Alkoholate der Formel (I b) sowie die Umsetzung mit Verbindungen der Formel (VIII) in einem Zweiphasensystem, wie beispielsweise wäßrige Natron- oder Kalilauge/Toluol oder Methylenchlorid, unter Zusatz von 0,01 bis 1 Mol eines Phasen-Transfer-Katalysators, wie beispielsweise einer Ammonium- oder

Phosphoniumverbindung, durchgeführt, wobei in der organischen Phase oder an der Grenzfläche die Alkoholate mit den in der organischen Phase befindlichen Halogenverbindungen umgesetzt werden.

Die Bis-azolyl-Derivate der Formel (I) können in Säureadditions-Salze bzw. Metallsalz-komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Stoffe eignen sich insbesondere zur Bekämpfung von Getreide- und Reiskrankheiten. So lassen sich Mehltau- und Rost-Krankheiten, Puccinia recondita, Pyrenophora teres, Cochliobolus sativus, Erysiphe graminis und Septoria nodorum an Getreide sowie Pyricularia und Pellicularia an Reis besonders gut bekämpfen.

Außerdem können sie sehr gut gegen Sphaerotheca fuliginea an Gurken, Venturia inaequalis an Äpfeln, Uromyces appendiculatus an Bohnen und Cercospora canescens an Bohnen verwendet werden.

Ferner zeichnen sich die erfindungsgemäß verwendbaren Stoffe auch durch herbizide und pflanzenwuchsregulierende Eigenschaften aus.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organisohem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Baor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw.

Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäß verwendbaren Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am

Wirkungsort erforderlich.

Bein Einsatz der erfindungsgemäß verwendbaren Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäß verwendbaren Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäß verwendbaren Wirkstoffe geht aus den nachfolgenden Beispielen hervor.

## Herstellungsbeispiele

### Beispiel 1

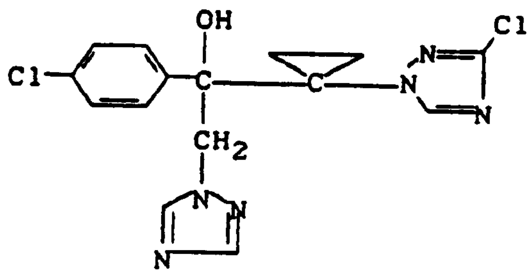

(I-1)

In ein Gemisch aus 15,6 g (0,276 Mol) 1,2,4-Triazol und 1,7 g (0,015 Mol) Kalium-tert.-butylat in 40 ml absolutem Dimethylformamid wird bei 80°C unter Rühren eine Lösung von 21 g (0,071 Mol) 2-(4-Chlorphenyl)-2-[1-(3-chlor-1,2,4-triazol-1-yl)-cyclopropyl]-oxiran in 30 ml absolutem Dimethylformamid eingetropft. Das Reaktionsgemisch wird weitere 6 Stunden bei 100°C gerührt und danach durch Abziehen des Lösungsmittels unter vermindertem Druck eingeengt. Der verbleibende Rückstand wird in einem Gemisch aus Essigsäureethylester/Toluol aufgenommen, mit Wasser gewaschen, über Natriumsulfat getrocknet und unter vermindertem Druck eingeengt. Der Rückstand wird mit einem Gemisch aus Dichlormethan/Ethanol = 98 : 2 als Laufmittel an Kieselgel chromatographiert. Durch Eindampfen des Eluates erhält man 11,5 g 1-(4-Chlorphenyl)-1-[1-(3-chlor-1,2,4-triazol-1-yl)cyclopropyl]-2-(1,2,4-triazol-1-yl)-ethan-1-ol in Form einer Festsubstanz vom Schmelzpunkt 156°C.

### Herstellung der Ausgangssubstanzen

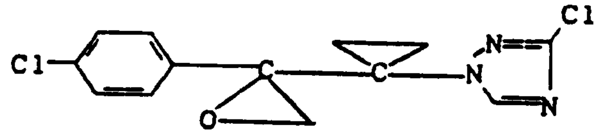

(V-1)

In ein Gemisch aus 2,5 g (83 mMol) Natriumhydrid (80-prozentig) und 17,6 g (80 mMol) Trimethylsulfoxoniumiodid werden bei 10°C unter Rühren unter Stickstoffatmosphäre 60 ml absolutes Dimethylsulfoxid zugetropft. Man rührt eine Stunde bei 20°C nach und gibt anschließend bei 10°C eine Lösung von 20 g (71 mMol) 1-(4-Chlorbenzoyl)-1-(3-chlor-1,2,4-triazol-1-yl)-cyclopropan in 30 ml absolutem Dimethylsulfoxid hinzu. Nach 48-stündigem Rühren bei 20°C wird das Reaktionsgemisch noch 1 Stunde auf 40°C erwärmt und dann auf Wasser gegossen. Man extrahiert das Gemisch mehrfach mit Essigsäurethylester, wäscht die vereinigten organischen Phasen mit Wasser, trocknet über Natriumsulfat und engt durch Abziehen des Lösungsmittels unter vermindertem Druck ein. Auf diese Weise erhält man 21 g (100 % der Theorie) an 2-(4-Chlorphenyl)-2-[1-(3-chlor-1,2,4-triazol-1-yl)-cyclopropyl]-oxiran in Form eines Öles.

$^1$H-NMR (200 MHz, $CDCl_3$): $\delta$ = 0,8 - 1,4 (m, 4H);
2,92 (d, 1H); 3,15 (d, 1H);
7,10 (d, 2H); 7,38 (d, 2H);
7,75. (s, 1H).

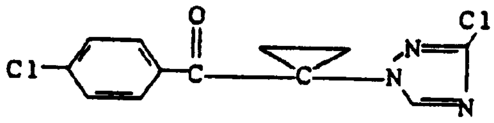

(II-1)

Ein Gemisch aus 34 g (246 mMol) Kaliumcarbonat und 35 g (338 mMol) 3-Chlor-1,2,4-triazol in 130 ml Aceton wird unter Rückfluß erhitzt. Dazu tropft man eine Lösung von 50 g (169 mMol) 2-Brom-4-chlor-1-(4-chlorphenyl)-butan-1-on in 60 ml Aceton. Man kocht weitere 8 Stunden unter Rückfluß, saugt dann vom festen Rückstand ab und zieht das Lösungsmittel unter vermindertem Druck ab. Der verbleibende Rückstand wird in Essigsäure-ethylester aufgenommen. Die Lösung wird mit Wasser gewaschen, über Natriumsulfat getrocknet und unter vermindertem Druck eingeengt. Der verbleibende Rückstand wird mit Dichlormethan als Laufmittel an Kieselgel chromatographiert. Durch Eindampfen des Eluates erhält man 42,9 g (90 % der Theorie) an 1-(4-Chlorbenzoyl)-1-(3-chlor-1,2,4-triazol-1-yl)-cyclopropan in Form einer Festsubstanz vom Schmelzpunkt 85°C.

Beispiel 2

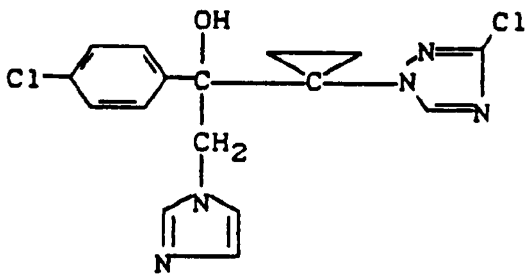

(I-2)

Unter Stickstoffatmosphäre wird ein Gemisch aus 13 g (191 mMol) Imidazol und 1 g (9 mMol) Kalium-tert.-butylat in 100 ml Acetonitril unter Rückfluß erhitzt. Dazu wird eine Lösung von 18 g (61 mMol) 2-(4-Chlorphenyl)-2-[1-(3-chlor-1,2,4-triazol-1-yl)-cyclopropyl]-oxiran in 30 ml Acetonitril getropft. Das Reaktionsgemisch wird weitere 10 Stunden unter Rückfluß erhitzt und dann durch Abziehen des Lösungsmittels unter vermindertem Druck eingeengt. Der verbleibende Rückstand wird in Essigsäureethylester aufgenommen und mit Wasser gewaschen. Die organische Phase wird nach dem Trocknen über Natriumsulfat durch Abziehen des Lösungsmittel unter vermindertem Druck eingeengt. Der verbleibende Rückstand wird mit einem Gemisch aus Dichlormethan/Ethanol = 90 : 10 als Laufmittel an Kieselgel chromatographiert. Durch Einengen des Eluates erhält man 7,1 g (32 % der Theorie) an 1-(4-Chlorphenyl)-1-[1-(3-chlor-1,2,4-triazol-1-yl)-cyclopropyl]-2-(imidazol-1-yl)-ethan-1-ol in Form einer Festsubstanz vom Schmelzpunkt 231°C.

Nach den zuvor angegebenen Methoden werden auch die in den folgenden Beispielen aufgeführten Stoffe hergestellt.

Beispiel 3

1-[1-(3-Chlor-1,2,4-triazol-1-yl)-cyclopropyl]-1-phenyl-2-(1,2,4-triazol-1-yl)-ethan-1-ol

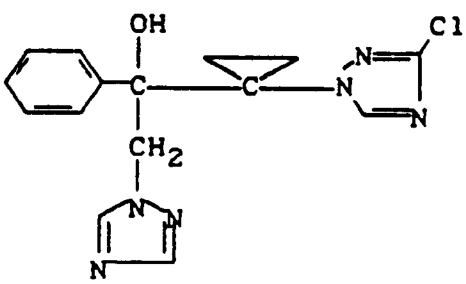

(I-3)

Fp.: 134°C

Beispiel 4

1-[1-(3-Chlor-1,2,4-triazol-1-yl)-cyclopropyl]-1-(4-fluorphenyl)-2-(1,2,4-triazol-1-yl)-ethan-1-ol

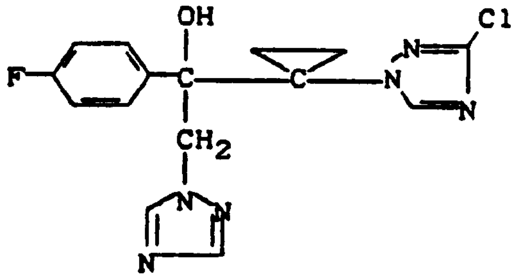

(I-4)

Fp.: 116°C

Beispiel 5

1-[1-(3-Chlor-1,2,4-triazol-1-yl)-cyclopropyl]-1-(4-methylphenyl)-2-(1,2,4-triazol-1-yl)-ethan-1-ol

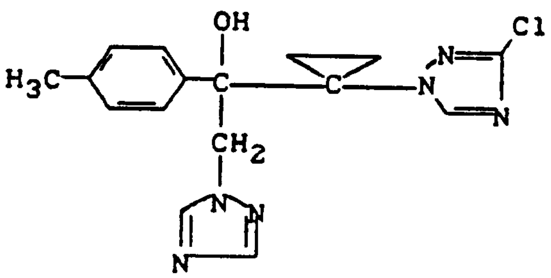

(I-5)

Fp.: 119°C

Beispiel 6

1-(4-Biphenyl)-1-[1-(3-Chlor-1,2,4-triazol-1-yl)-cyclopropyl]-2-(1,2,4-triazol-1-yl)-ethan-1-ol

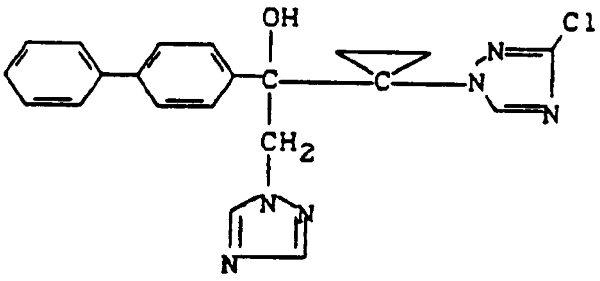

(I-6)

Fp.: 150°C

Beispiel 7

1-[1-(3-Chlor-1,2,4-triazol-1-yl)-cyclopropyl]-1-(2,4-difluorphenyl)-2-(1,2,4-triazol-1-yl)-ethan-1-ol

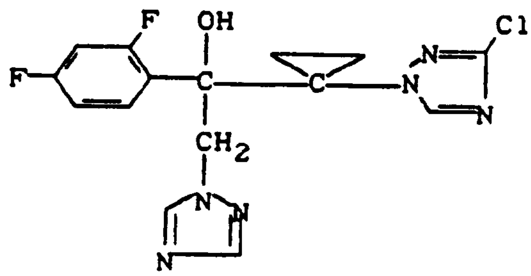

(I-7)

Fp.: 114°C

Beispiel 8

1-[1-(3-Chlor-1,2,4-triazol-1-yl)-cyclopropyl]-1-(3,4-difluorphenyl)-2-(1,2,4-triazol-1-yl)-ethan-1-ol

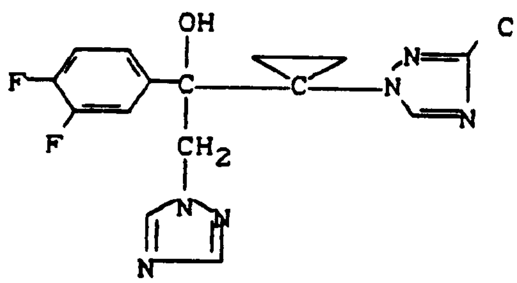

(I-8)

Fp.: 106°C

Beispiel 9

1-(3-Chlor-1,2,4-triazol-1-yl)-1-(4-fluorbenzoyl)-cyclopropan

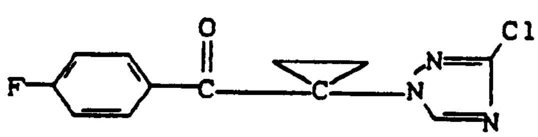

(I-2)

Fp.: 84°C

Verwendungsbeispiele

Beispiel A

Pyricularia-Test (Reis) /protektiv
Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Antrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalles.

Wirkstoffe, Wirkstoffkonzentrationen und Versuchsergebnisse gehen aus der folgenden Tabelle hervor.

Tabelle A

Pyricularia-Test (Reis) / protektiv

| Wirkstoffe | Wirkstoff-konzentration in % | Wirkungsgrad in % der unbehandelten Kontrolle |
|---|---|---|
| bekannt aus EP-OS 0 180 838: | | |
| (A) 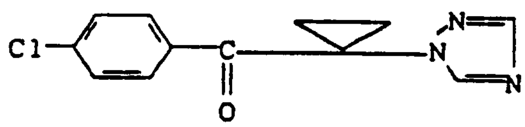 | 0,025 | 0 |
| (B) 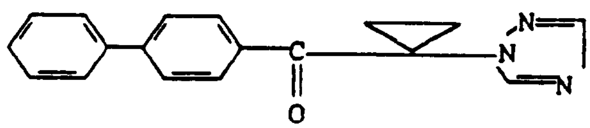 | 0,025 | 23 |
| (C) 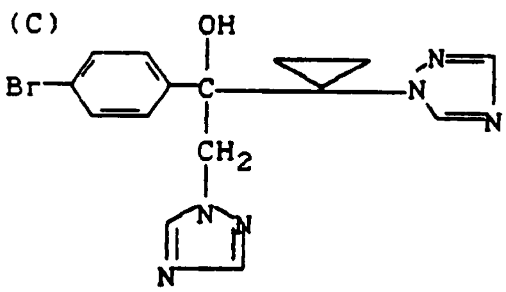 | 0,025 | 10 |
| erfindungsgemäß: | | |
| (I-1) 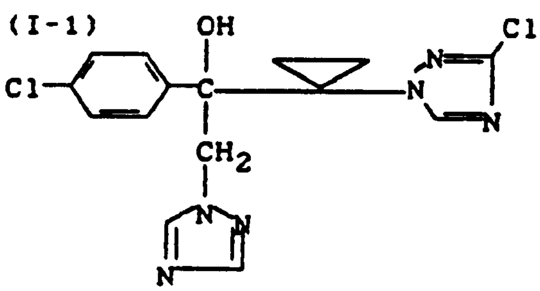 | 0,025 | 80 |

Tabelle A (Fortsetzung)

Pyricularia-Test (Reis) / protektiv

| Wirkstoffe | Wirkstoffkonzentration in % | Wirkungsgrad in % der unbehandelten Kontrolle |
|---|---|---|
| (I-4) 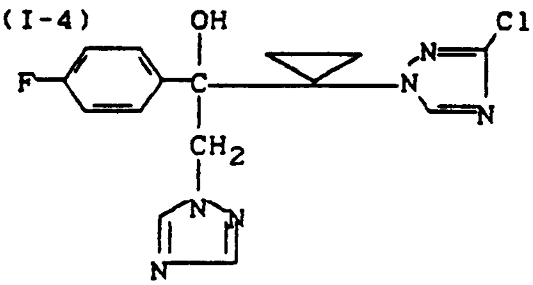 | 0,025 | 86 |
| (I-3) 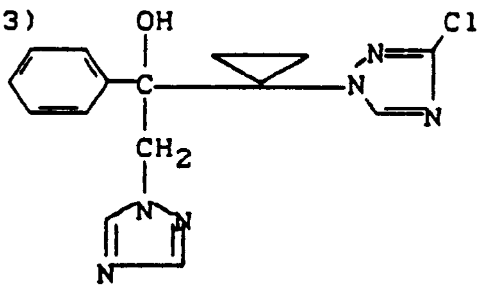 | 0,025 | 80 |
| (I-8) 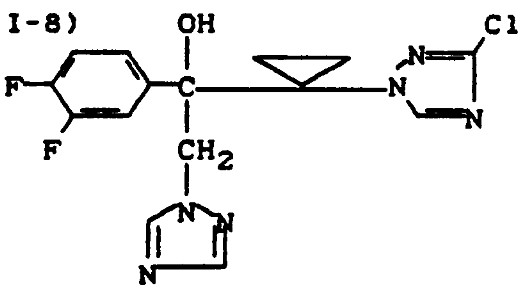 | 0,025 | 80 |

Tabelle A (Fortsetzung)

Pyricularia-Test (Reis) / protektiv

| Wirkstoffe | Wirkstoffkonzentration in % | Wirkungsgrad in % der unbehandelten Kontrolle |
|---|---|---|
| (I-5) 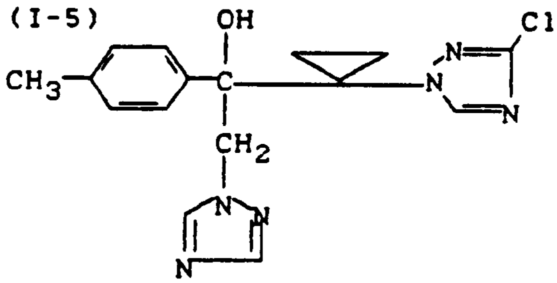 | 0,025 | 70 |
| (I-6) 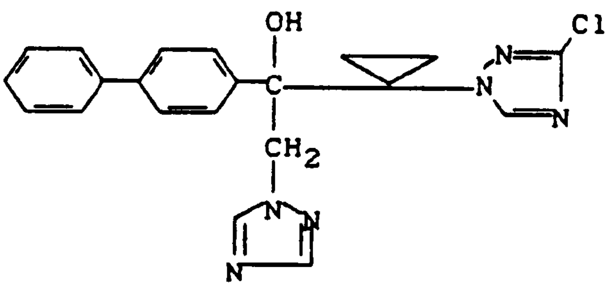 | 0,025 | 80 |

Beispiel B

Pyricularia-Test (Reis) / systemisch
Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei 100 % rel. Luftfeuchtigkeit und 25°C bis zur Auswertung.

4 Tage nach der Inokulation erfolgt die Auswertung des Krankheitsbefalles.

Wirkstoffe, Wirkstoffkonzentrationen und Versuchsergebnisse gehen aus der folgenden Tabelle hervor.

Tabelle B

Pyricularia-Test (Reis) / systemisch

| Wirkstoffe | Aufwandmenge in mg Wirkstoff pro 100 $cm^2$ | Wirkungsgrad in % der unbehandelten Kontrolle |
|---|---|---|
| bekannt aus EP-OS 0 180 838: | | |
| (A) 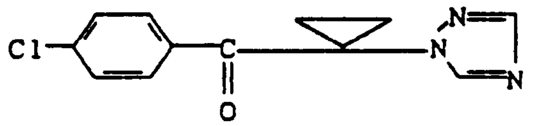 | 100 | 20 |
| (B) 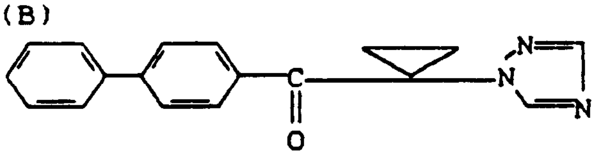 | 100 | 0 |
| (C) 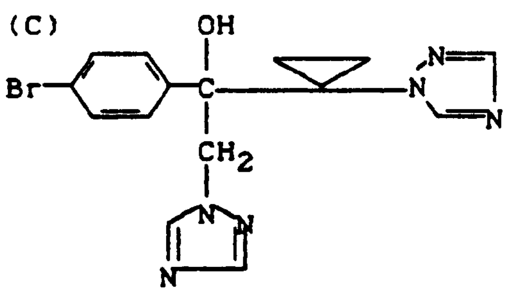 | 100 | 50 |

Tabelle B (Fortsetzung)

Pyricularia-Test (Reis) / systemisch

| Wirkstoffe | Aufwandmenge in mg Wirkstoff pro 100 $cm^2$ | Wirkungsgrad in % der unbehandelten Kontrolle |
|---|---|---|
| erfindungsgemäß: | | |
| (I-1) 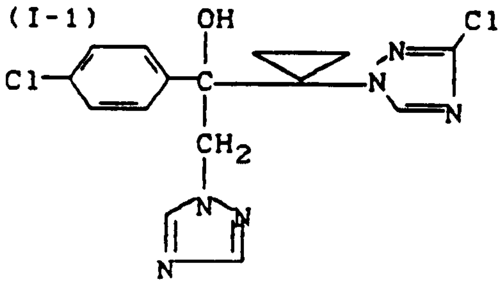 | 100 | 90 |
| (I-4) 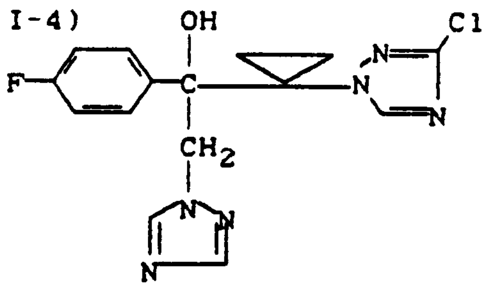 | 100 | 100 |
| (I-3) 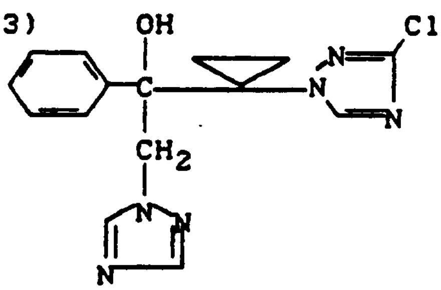 | 100 | 90 |

Tabelle B (Fortsetzung)

Pyricularia-Test (Reis) / systemisch

| Wirkstoffe | Aufwandmenge in mg Wirkstoff pro 100 $cm^2$ | Wirkungsgrad in % der unbehandelten Kontrolle |
|---|---|---|
| (I-8) 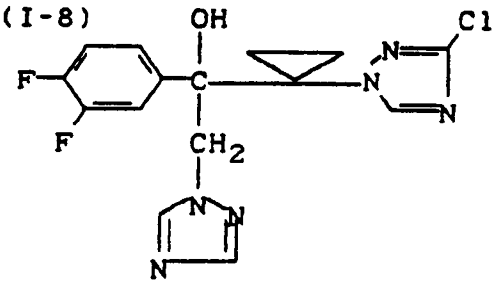 | 100 | 100 |
| (I-5) 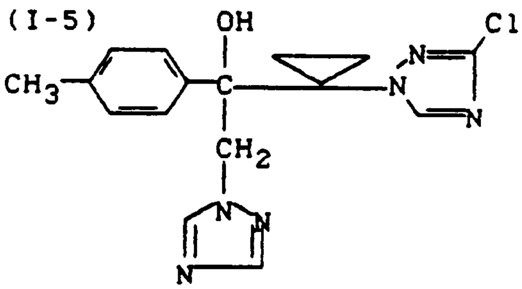 | 100 | 100 |
| (I-6) 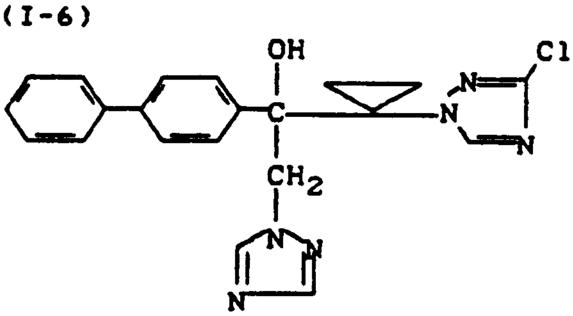 | 100 | 100 |

Beispiel C

Cochliobolus sativus-Test (Gerste) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespüht man junge Pflanzen mit der Wirkstoffzubereitung taufeucht. Nach dem Antrocknen des Spritzbelages werden die Pflanzen mit einer Sporensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Wirkstoffe, Wirkstoffkonzentrationen und Versuchsergebnisse gehen aus der folgenden Tabelle hervor.

## Tabelle C

Cochliobolus sativus-Test (Gerste) / protektiv

| Wirkstoffe | Wirkstoffkonzentration in in der Spritzbrühe in Gew.-% | Wirkungsgrad in % der unbehandelten Kontrolle |
|---|---|---|
| bekannt aus EP-OS 0 180 838: | | |
| (D) 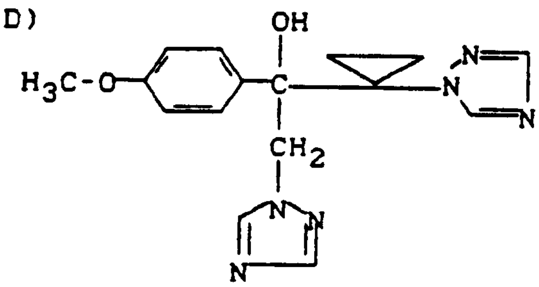 | 0,025 | 0 |
| (E) 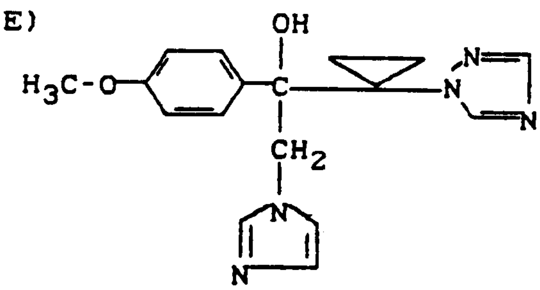 | 0,025 | 11 |
| (F) 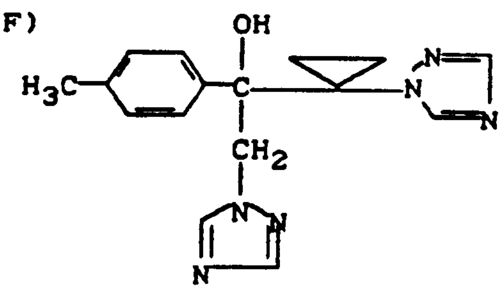 | 0,025 | 25 |

Tabelle C (Fortsetzung)

Cochliobolus sativus-Test (Gerste) / protektiv

| Wirkstoffe | Wirkstoffkonzentration in in der Spritzbrühe in Gew.-% | Wirkungsgrad in % der unbehandelten Kontrolle |
|---|---|---|
| (G) 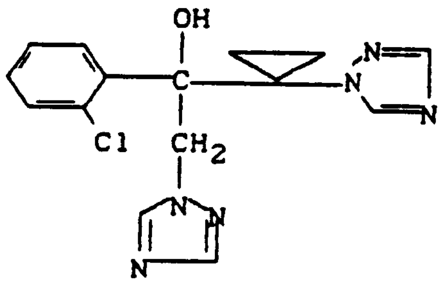 | 0,025 | 0 |
| (H) 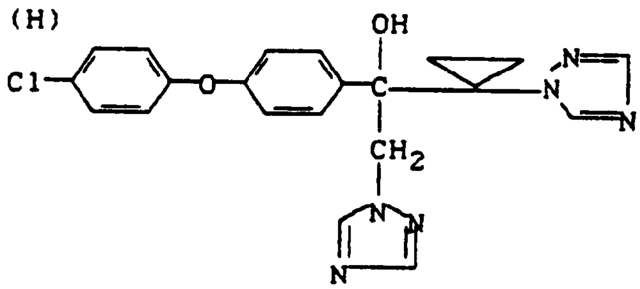 | 0,025 | 0 |
| (J) 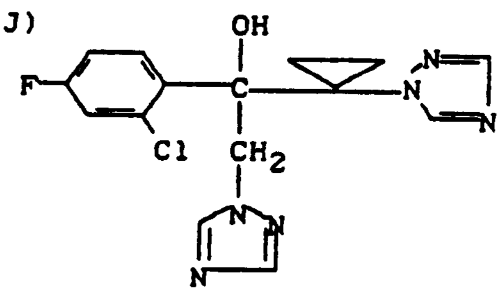 | 0,025 | 0 |

Tabelle C (Fortsetzung)

Cochliobolus sativus-Test (Gerste) / protektiv

| Wirkstoffe | Wirkstoffkonzentration in in der Spritzbrühe in Gew.-% | Wirkungsgrad in % der unbehandelten Kontrolle |
|---|---|---|
| (K) 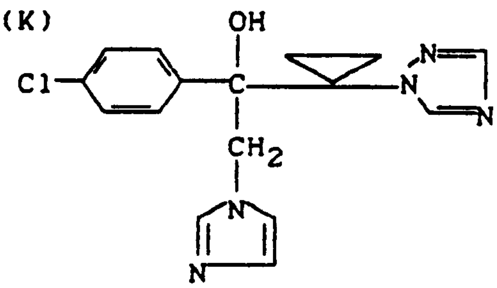 | 0,025 | 25 |
| (L) 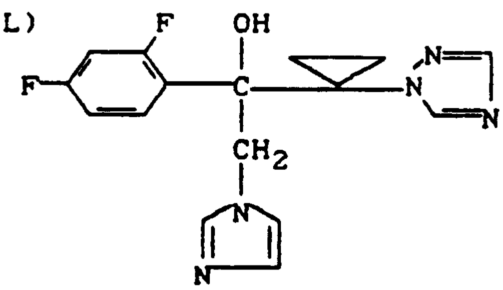 | 0,025 | 0 |

Tabelle C (Fortsetzung)

Cochliobolus sativus-Test (Gerste) / protektiv

| Wirkstoffe | Wirkstoffkonzentration in in der Spritzbrühe in Gew.-% | Wirkungsgrad in % der unbehandelten Kontrolle |
|---|---|---|
| erfindungsgemäß: | | |
| (I-6) 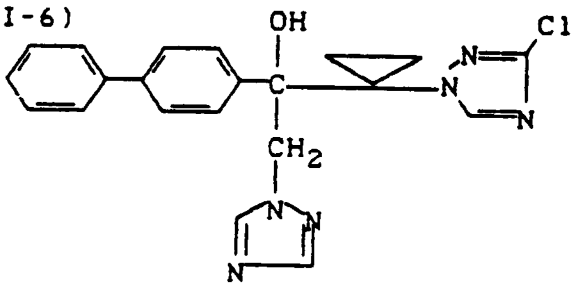 | 0,025 | 81 |
| (I-4) 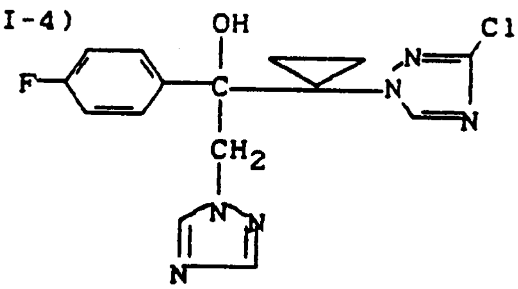 | 0,025 | 83 |

Beispiel D

Erysiphe-Test (Gerste) /protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit besprüht man junge Pflanzen mit der Wirkstoffzubereitung taufeucht. Nach dem Antrocknen des Spritzbelages werden die Pflanzen mit Sporen von Erysiphe gramins f.sp. hordei bestäubt.

Die Pflanzen werden in einem Gewächshaus bei einer Temperatur von ca. 20°C und einer rel. Luftfeuchtigkeit von ca. 80 % aufgestellt, um die Entwicklung von Mehltaupusteln zu begünstigen.

7 Tage nach der Inokulation erfolgt die Auswertung.

Wirkstoffe, Wirkstoffkonzentrationen und Versuchsergebnisse gehen aus der folgenden Tabelle hervor.

Tabelle D

Erysiphe-Test (Gerste) / protektiv

| Wirkstoffe | Wirkstoffkonzentration in in der Spritzbrühe in Gew.-% | Wirkungsgrad in % der unbehandelten Kontrolle |
|---|---|---|
| bekannt aus EP-OS 0 180 838: | | |
| (D) 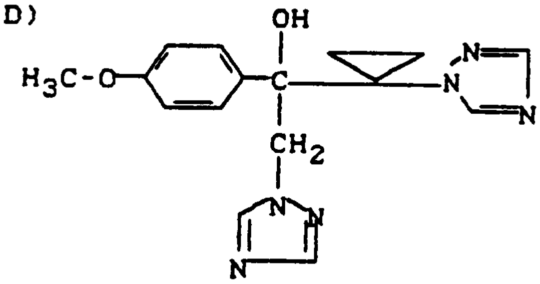 | 0,025 | 25 |
| (M) 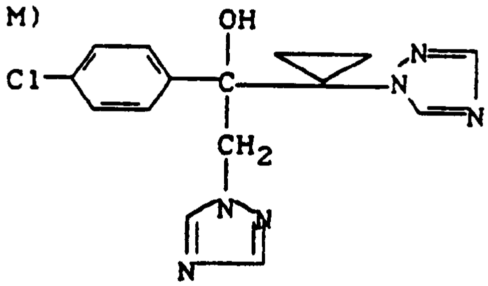 | 0,025 | 25 |
| (N) 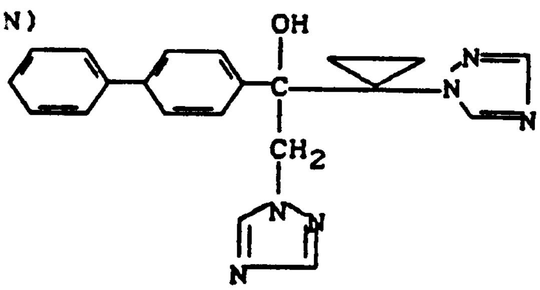 | 0,025 | 34 |

Tabelle D (Fortsetzung)

Erysiphe-Test (Gerste) / protektiv

| Wirkstoffe | Wirkstoffkonzentration in in der Spritzbrühe in Gew.-% | Wirkungsgrad in % der unbehandelten Kontrolle |
|---|---|---|
| erfindungsgemäß: | | |
| (I-6) 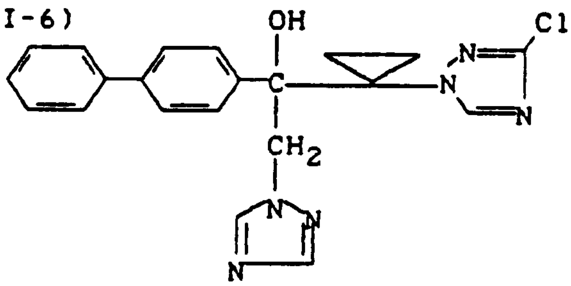 | 0,025 | 100 |
| (I-1) 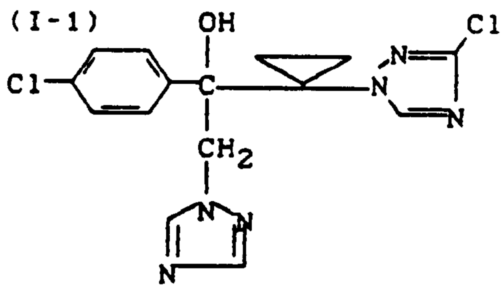 | 0,025 | 100 |
| (I-4) 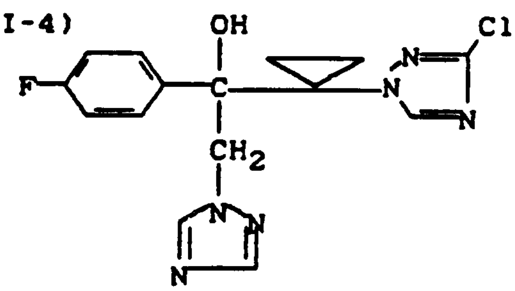 | 0,025 | 100 |

Tabelle D (Fortsetzung)

Erysiphe-Test (Gerste) / protektiv

| Wirkstoffe | Wirkstoffkonzentration in in der Spritzbrühe in Gew.-% | Wirkungsgrad in % der unbehandelten Kontrolle |
|---|---|---|
| (I-3) 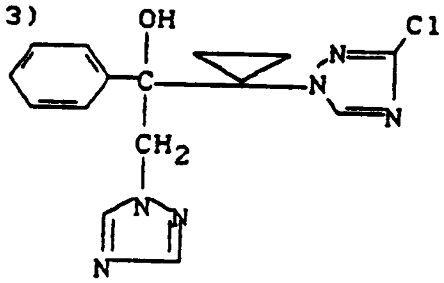 | 0,025 | 88 |
| (I-7) 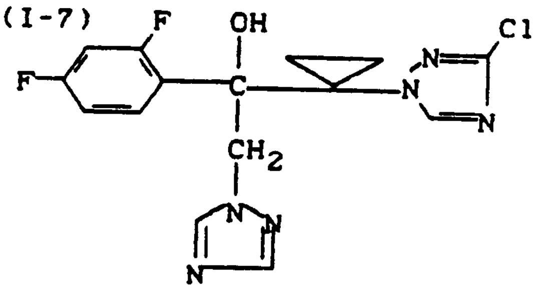 | 0,025 | 100 |
| (I-8) 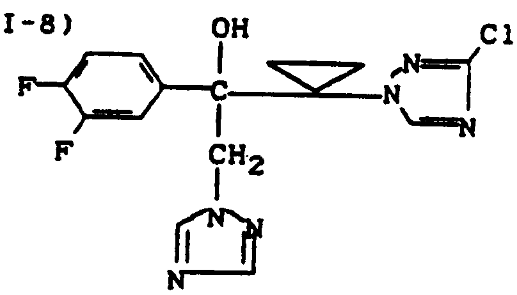 | 0,025 | 88 |

Tabelle D (Fortsetzung)

Erysiphe-Test (Gerste) / protektiv

| Wirkstoffe | Wirkstoffkonzentration in in der Spritzbrühe in Gew.-% | Wirkungsgrad in % der unbehandelten Kontrolle |
|---|---|---|
| (I-5) 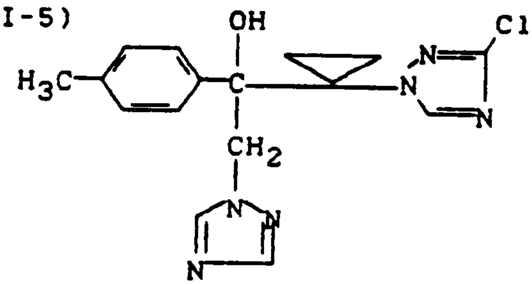 | 0,025 | 100 |
| (I-2) 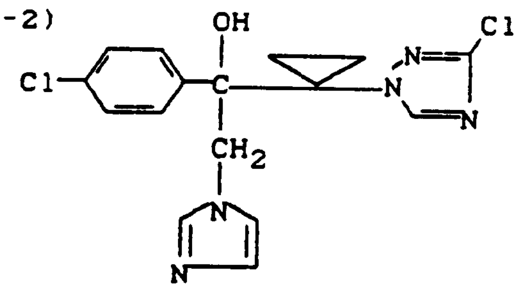 | 0,025 | 75 |

**Patentansprüche**

1. Fungizide Mittel, gekennzeichnet durch einen Gehalt an mindestens einem substituierten Bis-azolyl-Derivat der Formel

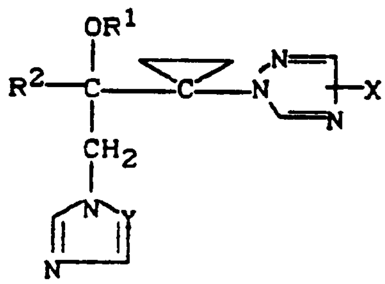

(I)

in welcher

$R^1$ - für Wasserstoff steht, oder

- für geradkettiges oder verzweigtes Alkyl mit bis zu 8 Kohlenstoffatomen steht, das gegebenenfalls durch Aryl substituiert ist, welches seinerseits durch Halogen, $C_1$-$C_8$-Alkyl, $C_1$-$C_8$-Alkoxy, $C_1$-$C_8$-Alkylthio, $C_1$-$C_8$-Halogenalkyl, $C_1$-$C_8$-Halogenalkoxy, $C_1$-$C_8$-Halogenalkylthio oder durch

Phenyl substituiert ist, das gegebenenfalls bis zu 4-fach gleich oder verschieden durch Halogen substituiert ist oder
- für geradkettiges oder verzweigtes Alkenyl oder Alkinyl mit bis zu 8 Kohlenstoffatomen steht oder
- für Trialkylsilyl mit bis zu 8 Kohlenstoffatomen im Alkylteil steht oder
- für Alkylcarbonyl mit bis zu 8 Kohlenstoffatomen im Alkylteil steht,

$R^2$ - für Aryl mit 6 bis 10 Kohlenstoffatomen steht, das gegebenenfalls bis zu 4-fach gleich oder verschieden substituiert ist durch Halogen, $C_1$-$C_8$-Alkyl, $C_1$-$C_8$-Alkoxy, $C_1$-$C_8$-Alkylthio, Halogenalkyl mit 1 bis 8 Kohlenstoffatomen und 1 bis 5 gleichen oder verschiedenen Halogenatomen, Halogenalkoxy mit 1 bis 8 Kohlenstoffatomen und 1 bis 5 gleichen oder verschiedenen Halogenatomen, Halogenalkylthio mit 1 bis 8 Kohlenstoffatomen und 1 bis 5 gleichen oder verschiedenen Halogenatomen, gegebenenfalls ein-bis dreifach durch Halogen substituiertes Phenyl, gegebenenfalls ein- bis dreifach durch Halogen substituiertes Phenoxy und/oder durch gegebenenfalls ein- bis dreifach durch Halogen substituiertes Phenylthio, oder

$R^2$ - für einen 5- oder 6-gliedrigen Heterocyclus steht, der ein oder mehrere Heteroatome, wie Schwefel, Sauerstoff oder Stickstoff, enthalten kann und der gegebenenfalls durch Halogen, $C_1$-$C_8$-Alkyl, $C_1$-$C_8$-Alkoxy, $C_1$-$C_8$-Alkylthio, Halogenalkyl mit 1 bis 8 Kohlenstoffatomen und 1 bis 5 gleichen oder verschiedenen Halogenatomen, Halogenalkoxy mit 1 bis 8 Kohlenstoffatomen und 1 bis 5 gleichen oder verschiedenen Halogenatomen und/oder Halogenalkylthio mit 1 bis 8 Kohlenstoffatomen und 1 bis 5 gleichen oder verschiedenen Halogenatomen substituiert ist,

X - für Halogen steht,

und

Y - für ein Stickstoffatom oder für die CH-Gruppe steht,

bzw. an einem Säureadditions-Salz oder Metallsalz-Komplex eines substituierten Bis-azolyl-Derivates der Formel (I).

2. Verwendung von substituierten Bis-azolyl-Derivaten der Formel (I) gemäß Anspruch 1 sowie von deren Säureadditions-Salzen oder Metallsalz-Komplexen zur Bekämpfung von Pilzen.

3. Verfahren zur Bekämpfung von Pilzen, dadurch gekennzeichnet, daß man substituierte Bis-azolyl-Derivate der Formel (I) gemäß Anspruch 1 bzw. deren Säureadditions-Salze oder Metallsalz-Komplexe auf die Pilze und/oder deren Lebensraum ausbringt.

4. Verfahren zur Herstellung von fungiziden Mitteln, dadurch gekennzeichnet, daß man substituierte Bis-azolyl-Derivate der Formel (I) gemäß Anspruch 1 bzw. deren Säureadditions-Salze oder Metallsalz-Komplexe mit Streckmitteln und/oder oberflächenaktiven Stoffen vermischt.

## Claims

1. Fungicidal agents, characterized in that they contain at least one substituted bis-azolyl derivative of the formula

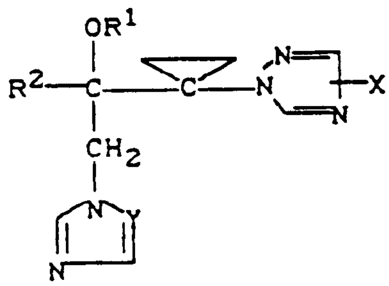

(I)

in which

$R^1$ - represents hydrogen, or
- represents straight-chain or branched alkyl which has up to 8 carbon atoms and is optionally substitued by aryl which, in turn, is substituted by halogen, $C_1$-$C_8$-alkyl, $C_1$-$C_8$-alkoxy, $C_1$-$C_8$-al-

kylthio, $C_1$-$C_8$-halogenoalkyl, $C_1$-$C_8$-halogenoalkoxy, $C_1$-$C_8$-halogenoalkylthio or by phenyl which is optionally up to tetrasubstituted by identical or different halogen substituents, or
- represents straight-chain or branched alkenyl or alkinyl, each of which has up to 8 carbon atoms, or
- represents trialkylsilyl having up to 8 carbon atoms in the alkyl moiety, or
- represents alkylcarbonyl having up to 8 carbon atoms in the alkyl moiety,

$R^2$ - represents aryl which has 6 to 10 carbon atoms and which is optionally up to tetrasubstituted by identical or different substituents from the series comprising halogen, $C_1$-$C_8$-alkyl, $C_1$-$C_8$-alkoxy, $C_1$-$C_8$-alkylthio, halogenoalkyl having 1 to 8 carbon atoms and 1 to 5 identical or different halogen atoms, halogenoalkoxy having 1 to 8 carbon atoms and 1 to 5 identical or different halogen atoms, halogenoalkylthio having 1 to 8 carbon atoms and 1 to 5 identical or different halogen atoms, phenyl which is optionally monosubstituted to trisubstituted by halogen, phenoxy which is optionally monosubstituted to trisubstituted by halogen, and/or phenylthio which is optionally monosubstituted to trisubstituted by halogen, or

$R^2$ - represents a 5- or 6-membered heterocyclic ring which can contain one or more hetero atoms, such as sulphur, oxygen or nitrogen, and which is optionally substituted by halogen, $C_1$-$C_8$-alkyl, $C_1$-$C_8$-alkoxy, $C_1$-$C_8$-alkylthio, halogenoalkyl having 1 to 8 carbon atoms and 1 to 5 identical or different halogen atoms, halogenoalkoxy having 1 to 8 carbon atoms and 1 to 5 identical or different halogen atoms, and/or halogenoalkylthio having 1 to 8 carbon atoms and 1 to 5 identical or different halogen atoms,

X - represents halogen,
and

Y - represents a nitrogen atom or the CH group,

or an acid addition salt or metal salt complex of a substituted bis-azolyl derivative of the formula (I).

2. Use of substituted bis-azolyl derivatives of the formula (I) according to Claim 1 and of their acid addition salts or metal salt complexes for combating fungi.

3. Process for combating fungi, characterized in that substituted bis-azolyl derivatives of the formula (I) according to Claim 1, or their acid addition salts or metal salt complexes, are applied to the fungi and/or their environment.

4. Process for the preparation of fungicidal agents, characterized in that substituted bis-azolyl derivatives of the formula (I) according to Claim 1, or their acid addition salts or metal salt complexes, are mixed with extenders and/or surface-active substances.

## Revendications

1. Compositions fongicides, caractérisées par une teneur en au moins un dérivé bis-azolylique substitué de formule

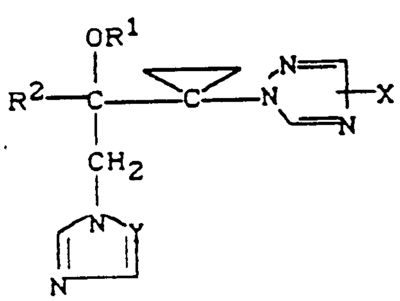

(I)

dans laquelle

$R^1$ - représente l'hydrogène, ou
- un groupe alkyle à chaîne droite ou ramifiée ayant jusqu'à 8 atomes de carbone, qui est substitué, le cas échéant, par un radical aryle, lequel est substitué de son côté par un halogène, un radical alkyle en $C_1$ à $C_8$, alkoxy en $C_1$ à $C_8$, alkylthio en $C_1$ à $C_8$, halogénalkyle en $C_1$ à $C_8$, ha-

logénalkoxy en $C_1$ à $C_8$, halogénalkylthio en $C_1$ à $C_8$ ou par un radical phényle qui porte, le cas échéant, jusqu'à 4 subtituants halogéno identiques ou différents, ou bien
- un groupe alcényle ou alcynyle à chaîne droite ou ramifiée ayant jusqu'à 8 atomes de carbone, ou bien
- un groupe trialkylsilyle ayant jusqu'à 8 atomes de carbone dans la partie alkyle, ou bien
- un groupe alkylcarbonyle ayant jusqu'à 8 atomes de carbone dans la partie alkyle,

$R^2$ - est un groupe aryle ayant 6 à 10 atomes de carbone, qui porte, le cas échéant, jusqu'à 4 substituants, identiques ou différents, halogéno, alkyle en $C_1$ à $C_8$, alkoxy en $C_1$ à $C_8$, alkylthio en $C_1$ à $C_8$, halogénalkyle ayant 1 à 8 atomes de carbone et 1 à 5 atomes d'halogènes identiques ou différents, halogénalkoxy ayant 1 à 8 atomes de carbone et 1 à 5 atomes d'halogènes identiques ou différents, halogénalkylthio ayant 1 à 8 atomes de carbone et 1 à 5 atomes d'halogènes identiques ou différents, phényle portant, le cas échéant, 1 à 3 substituants halogéno, phénoxy portant, le cas échéant, 1 à 3 substituants halogéno et/ou phénylthio portant, le cas échéant, 1 à 3 substituants halogéno, ou bien

$R^2$ - est un hétérocycle pentagonal ou hexagonal qui peut comporter un ou plusieurs hétéro-atomes tels que soufre, oxygène ou azote et qui est substitué, le cas échéant, par un halogène, un groupe alkyle en $C_1$ à $C_8$, alkoxy en $C_1$ à $C_8$, alkylthio en $C_1$ à $C_8$, halogénalkyle ayant 1 à 8 atomes de carbone et 1 à 5 atomes d'halogènes identiques ou différents, halogénalkoxy ayant 1 à 8 atomes de carbone et 1 à 5 atomes d'halogènes identiques ou différents et/ou halogénalkylthio ayant 1 à 8 atomes de carbone et 1 à 5 atomes d'halogènes identiques ou différents,

X - est un halogène,

et

Y - est un atome d'azote ou le groupoe CH,

ou, respectivement en un sel d'addition d'acide ou un complexe de sel métallique d'un dérivé bis-azolylique substitué de formule (I).

**2.** Utilisation de dérivés bis-azolyliques substitués de formule (I) suivant la revendication 1 ainsi que de leurs sels d'addition d'acides ou de leurs complexes de sels métalliques pour combattre des champignons.

**3.** Procédé pour combattre des champignons, caractérisé en ce qu'on applique des dérivés bis-azolyliques substitués de formule (I) suivant la revendication 1 ou leurs sels d'addition d'acides ou leurs complexes de sels métalliques sur les champignons et/ou sur leur milieu.

**4.** Procédé de préparation de compositions fongicides, caractérisé en ce qu'on mélange des dérivés bis-azolyliques substitués de formule (I) suivant la revendication 1 ou leurs sels d'addition d'acides ou leurs complexes de sels métalliques avec des diluants et/ou des agents tensio-actifs.